# EUROPEAN PATENT SPECIFICATION

(11) **EP 0 658 109 B1**
(45) Date of publication and mention of the grant of the patent: **30.05.2001**
(21) Application number: 93921333.6
(22) Date of filing: 03.09.1993
(51) Int. Cl.: A61K 31/40, A61K 31/69, C07F 5/05, A61K 31/555, C07F 5/02, C07D 487/22

(54) **METALLO PORPHYRIN COMPOSITIONS**
METALLPORPHYRINZUSAMMENSETZUNGEN
COMPOSITIONS DE METALLOPORPHYRINE

(30) Priority: 03.09.1992 US 940095
(43) Date of publication of application: 21.06.1995
(73) Proprietor: THE REGENTS OF THE UNIVERSITY OF CALIFORNIA, Oakland, California 94612-3550 (US)
(72) Inventor: KAHL, Stephen, B., Portola Valley, CA 94028 (US); CRAIK, Charles, S., San Francisco, CA 94122 (US)
(74) Representative: Tiedtke, Harro, Dipl.-Ing.
(86) International application number: US9308317
(87) International publication number: WO9405285

(56) References cited:
- EP-A- 0 337 598
- WO-A-92/02242
- WO-A-92/09610
- US-A- 4 959 356
- US-A- 5 109 016
- US-A- 5 128 319
- J. MED. CHEM., vol. 35, no. 18, 1992, P3D016, pages 3426-3428, XP002022700 DECAMP, D.L. ET AL.: "Specific Inhibition of HIV-1 Protease by Boronated Porphyrins"
- PROC. NATL. ACAD. SCI USA, vol. 87, 1990, pages 6644-6648, XP000605236 DESJARLAIS, ET AL.: "Structure-based design of nonpeptide inhibitors specific for the human immunodeficiency virus 1 protease."

## Description

### Field of the Invention.

The present invention generally relates to porphyrins, and more particularly to porphyrins with hydrophobic glycol derivative substituents at pyrrole ring positions 2 and 4 and that are complexed with metals and to its use in the preparating of a medicament effective for the treatment of retroviral diseases.

### Background of the Invention.

U.S. Patent 4,959,356, issued September 25, 1990, inventors Miura and Gabel describe boronated porphyrin compounds for use in boron neutron capture theory (BNCT). Similar compounds are discussed in an article by Miura et al. *Tetrahedron Letters*, Vol. 31, No. 16, pp. 2247-2250 (1990). The boronated porphyrins described by Miura have vinyl carborane moieties that she reports as not being water soluble. Thus she must open the borane cages. But by opening those borane cages, one encounters significantly more toxicity for the compounds. Moreover, the resultant open-cage compounds are still not sufficiently water soluble to enable administration without the use of adjuvant substances (e.g., polyethylene glycol). Also the compounds which Miura et al. describe are (at most) 19% boron by weight in the physiologically useful (K⁺-salt) form. This is a disadvantage since limiting human doses may well be determined by the amount of porphyrin unit doses which may be tolerated.

In addition to neutron capture therapy (NCT) generally and boron neutron capture therapy (BNCT) more specifically, additional uses of porphyrins in cancer therapies are those therapeutic strategies generally referred to as photodynamic therapy (PDT). A review article by Delaney and Glatstein in *Comprehensive Therapy,* pp. 43-55 (May 1988) describes this therapeutic strategy where a light-activated photosynthesizer can interact with ground state molecular oxygen to yield reactive oxygen species (via singlet oxygen). Since porphyrins of many structural types localize in a wide variety of malignant tumors, this localization has formed the basis for treatment of at least 3000 patients in the United States alone (twice that worldwide) over the past several years through PDT. Complete response (disappearance of tumor or biopsy proven) has occurred in a high percentage of patients in relatively advanced stages of skin, bladder, and lung cancers, and cancers of the reproductive system through photodynamic therapy.

The rapid spread of human immunodeficiency virus (HIV), believed the causative agent of acquired immunodeficiency syndrome (AIDS), throughout the world has prompted an intense search for anti-retroviral therapeutics. Des Jarlais et al. have discussed some strategies for HIV inhibitors in their "Structure-Based Design of Non-Peptide Inhibitors Specific for the Human Immunodeficiency I Protease," *Proc*. *Natl*. *Acad*. *Sci*. *USA, 87,* pp. 6644-6648 (1990).

U.S. Patent 5,109,016, issued April 28, 1992, inventors Dixon et al., describes compositions said to inhibit replication of human immunodeficiency virus by porphyrin and certain porphyrin-like compounds. These compounds were tested for inhibition of reverse transcriptase as a screening method to determine inhibition of HIV; however, during the 8th International Conference on AIDS, held in late July, 1992, in Amsterdam, some presentations suggested that many drugs that showed initial promise against HIV-1 have been found to have serious shortcomings because HIV-1 rapidly becomes resistant to the so-called "non-nucleoside reverse transcriptase inhibitors." See, *C&EN*, *70*:34, pp. 26-31 (August 24, 1992). As an example of a non-nucleoside derivative that binds to a site other than the active site (non-competitive inhibition), see Pauwels et al., *Nature, 343*, pp. 470-474 (1990); however, resistance develops rapidly to this derivative.

Related to HIV treatments or prevention is U.S. Patent 5,128,319, issued July 7, 1992, which describes compositions said useful to immunize against HIV. The compositions have sequences that correspond to portions of a conserved domain of a HIV protein. Also, U.S. Patent 5,132,291, issued July 21, 1992, describes a method of enhancing the anti-viral effect of AZT.

AZT, which is presently being used in AIDS therapy, inhibits the retroviral enzyme reverse transcriptase, and is a "nucleoside" reverse transcriptase inhibitor. However, resistance to AZT develops in chronic treatments with the drug even though the drug does go to the active site of reverse transcriptase, as described by Larder and Kemp, *Science, 246*, pp. 1155-1158 (1989). It is only one of two drugs which have been fully approved for combating HIV-1 infections. Both zidovudine, which is 3'-azido-3'-deoxythymidine (AZT), and 2',3'-dideoxyinosie (DDI) are nucleoside analogs that interfere with HIV-1 reverse transcriptase, the enzyme that catalyzes the synthesis of a DNA copy of HIV-l's RNA genome. Another nucleoside analog, 2',3'-dideoxycytosine (DDC), has been shown to be effective against HIV-1 in some patients and has been approved for limited use.

A recent alternate approach has been attempted to target the retroviral protease. Thus, an experimental drug (designated "U-75875") is described by Ashorn et al., *PNAS*, *87,* pp. 7472-7476 (1990). However, this experimental drug has been found to be not very bioavailable, which may be due to poor absorption, to rapid clearing from the body, or from other causes.

EP-A-0 337 598 discloses the use of porphyrins and metalloporphyrins in the treatment of diseases caused by human immunodeficiency viruses. The porphyrin compounds may comprise substitutions in the pyrrol rings which are alkyl, alkenyl, hydroxyalkyl, carboxy or carboxyalkyl groups.

WO 92/02242 discloses the use of metalloporphyrins for the preparation of compositions for treating viral infections especially retroviral infections such as AIDS wherein the metalloporphyrins such as heme, organometallic porphyrins and mixtures thereof are the therapeutically active agents in the treatment.

However, there is still a strong need for the provision of new medicaments for treating retroviral infections which show a long lasting and increased activity without showing rapid resistance of the virus against the medicament and without showing toxicity.

### Summary of the Invention.

Porphyrin compounds of this invention have the structure 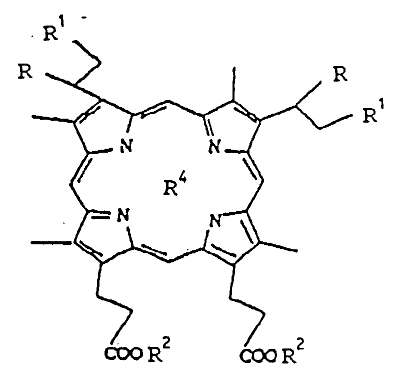 where each of R and R¹ is selected from ―H, ―OH, or 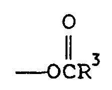 and at least one of R and R¹ is 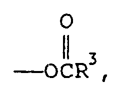 R³ is a carborane, R² is ―H, an alkyl, or an aryl, having 1 to about 7 carbon atoms, or a physiologically acceptable salt, and R⁴ is H, but more preferably is a metal, or a transition metal. The particularly preferred porphyrin compound is the tetrakiscarborane carboxylate ester of 2,4-(α,β-dihydroxylethyl)deutero-porphyrin IX complexed with manganese, copper, or cobalt.

The invention is further directed to the use of the above porphyrin compounds in the preparation of a medicament effective for the treatment of diseases caused by a virus that replicates through use of a protease comprising administrating said porphyrin compound or a physiologically acceptable salt thereof to a patient in a therapeutic amount effective to inhibit the protease.

### Brief Description of the Drawings.

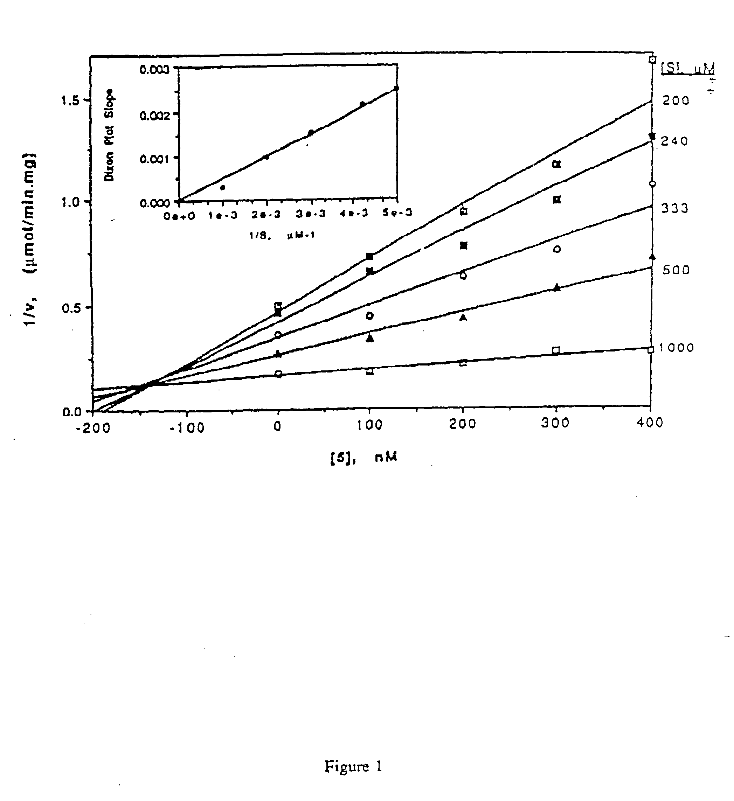

Figure 1 is a Dixon plot of inhibition by one inventive embodiment with an inhibition constant (Ki) of 140±25 nM, which is consistent with a competitive mode of inhibition and shows the strength of binding to HIV-1 protease.

### Detailed Description of the Preferred Embodiments.

Broadly, the invention relates to compositions and preparation methods for a therapeutically effective drug consisting of or built from a porphine precursor, whose basic structure is illustrated as Formula P:

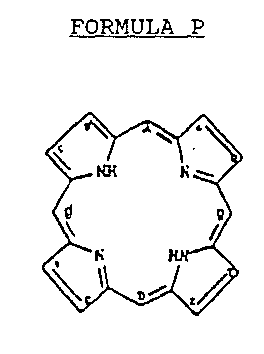

Porphine, of course, is the parent (or core) substance of the porphyrins, which have side chains and methyl groups substituted for some hydrogens in the porphine pyrrole rings. Compounds of the invention have substituents on at least two of the pyrrole rings of porphine at pyrrole ring positions 2 and 4. At least some of these substituents at pyrrole ring positions 2 and 4 preferably are hydrophobic derivatives of glycol.

Porphyrin compounds of the invention have the structure illustrated by Formula 1: 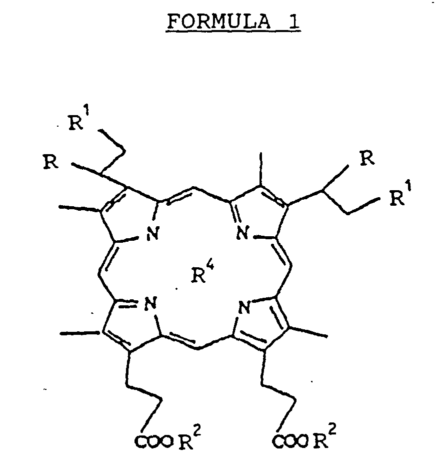 where each of R and R¹ is selected from ―H, ―OH, or 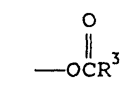 and at least one of R and R¹ is 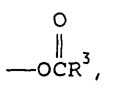 R³ is a carborane, R² is ―H, an alkyl, or an aryl, having 1 to about 7 carbon atoms, or a physiologically acceptable salt, and R⁴ is H, a metal, or a transition metal.

Particularly preferred embodiments of this invention are where R³ is a *closo*-carborane, and R⁴ is metal ion (e.g. Zn(II)), or a transition metal, such as Mn(III), Cu(II), or Co(II). The presence of a metal assists in stabilizing the molecules against, for example, sensitivity to light.

Compounds with boron cage systems in which one or more carbon atom is present as an integral part of an electron-delocalized boron framework are given the general name "carboranes", which term includes both closed polyhedra and open-cage structures. Carboranes are distinct from other organoboron species, such as the alkyl boranes, because the carbon(s) are part of the cage itself rather than present as a ligand. An early monograph on these electron-deficient boron cage compounds is by Grimes, *Carboranes,* Academic Press (1970), which describes nomenclature, structure, synthesis, and properties of carboranes, including those of interest for this invention. There are a series of stable 12-atom polyhedral boron cage systems that have been isolated in at least three isomeric forms. Two particularly preferred cage system isomers for this invention are the 1,2-C₂B₁₀H₁₂ isomer and the 1,7-C₂B₁₀H₁₂ isomer (the latter sometimes represented as "HCB₁₀H₁₀CH").

Particularly preferred for embodiments of the invention are wherein R³ is a closo-carborane, and most particularly is the 1,2-icosahedral isomer or 1,7-icosahedral isomer, whether substituted or unsubstituted. An illustrative substituent is, for example, a carboxyl group, which may be desirable to aid in solubilizing the porphyrin compound. The physical properties of the 1,2-icosahedral isomer and the 1,7-icosahedral isomer are similar.

The *closo*-carboranes are particularly preferred because the open-cage carboranes lead to significantly increased toxicity of what can otherwise be substantially non-toxic compounds. Closed-cage and open-cage systems are typically designated by the prefixes *"closo-"* and "nido-", respectively.

Conversion of the bis-glycol substituents to tetra-ester is surprising due to the size and steric hindrance considerations for preferred moieties. Where one desires to only obtain a di-ester carborane (such as with an R¹ at each of the 2 and 4 porphyrin ring positions as hydroxyl or hydrogen), then typical acylation agents, such as pyridine or trimethyl amine can be used; however, where one desires the tetra-ester, such as in preparing the particularly preferred embodiment we have coined as "BOPP", then the conversion of bis-glycol critically depends upon the use of p-dimethylaminopyridine (DMAP) as a hyper-acylation reagent.

The particularly preferred embodiment is especially useful where a stable, quite water soluble, substantially non-toxic compound with a large number of boron atoms (40) is desired. The large number of boron atoms is believed useful, for example, in treating solid tumors in combination with neutron capture therapy (NCT). In the potassium salt form, the BOPP embodiment is very water soluble and can be readily prepared in concentrations of at least about 100-200 mg/mL, yet the compound retains a high degree of lipophilicity. We also have designated this BOPP embodiment by the phrase "tetra-carborane carboxylate ester of 2,4-Di(α,β-dihydroxyethyl)deuteroporphyrin (IX)" or by "embodiment 1."

Embodiments can also be used for inhibition of retroviral aspartyl proteases *in vitro,* such as use of BOPP esterified with four molecules of 1,2-dicarbo*closo*-dodecaborane-carboxylic acid. Removal of all four carborane moieties substantially reduces inhibition of aspartyl proteases. However, we have found that removal of only two of the four carborane cages has little effect on binding with the HIV-1 and HIV-2 proteases. This suggests that only two of the four closo-carborane cages are responsible for most of the binding interaction. The metacarborane isomer binds approximately 60-fold less tightly, indicating that not only the presence of the carborane groups but also their isomeric conformation is important. Adding a methyl group to the unsubstituted carborane cage CH also substantially decreases the binding affinity.

The carborane cages appear to have a specific interaction with the HIV proteases, which results in high affinity between the molecule and enzymes. Replacement of the carborane cages with similarly sized, but less hydrophobic groups, such as benzoyl, adamantoyl, or even β-naphthoyl groups gives inhibitors with IC₅₀ values in the low micromolar range.

The inventive porphyrin molecules tend to have sensitivity to light, but can be stabilized by forming transition metal complexes. Although complexation with Co(II) or Cu(II) weakens binding about 10-fold, addition of Mn(III) has only a two-fold effect on inhibition, which indicates that the hexacoordinate Mn(III) may be able to make favorable ionic interactions with the enzyme.

While oral administration is preferred for compounds of the invention, i.v. or i.p. administration can be used. The preferred BOPP dipotassium salt ester embodiment is substantially water soluble and is thus suitable for oral administration. One can administer as a single portion, such as of 100 mg/kg, or administer in serial portions over a period of time as advised by an attending physician. The particularly preferred embodiment has been given to mice at doses as high as 200 mg/Kg with no apparent signs of morbidity or mortality. Mice receiving this embodiment at 100 mg/Kg by i.v. bolus have been exposed to peak plasma concentrations of almost 1 mM. This is well above the concentrations that are effective for *in vitro* antiproteoyl activity or *ex vivo* anti-viral activity. Thus, toxicity does not appear to be an impediment for practice of the invention. Yet further, when we performed experiments to determine specificity, we found that the particularly preferred embodiment was much more specific for HIV-1, HIV-2, and the simian retrovirus, SIV aspartyl proteases, than to the cellular aspartyl proteases such as renin and pepsin.

Porphyrin compositions used as described in accordance with this invention are substantially bioavailable. As will be exemplified hereinafter, *in vitro* effect of the various porphyrin derivatives on HIV-1 and HIV-2 protease activity was examined by monitoring the cleavage of a decapeptide substrate. Since many of the derivatives tested were insoluble in buffer alone at the concentrations necessary for the IC₅₀ determinations, 5% DMSO was used to increase solubility and to allow a fair comparison of the binding affinities of the various compounds. However, -preferred embodiments in accordance with this invention when used as inhibitors are soluble in aqueous solution, and these preferred embodiments were actually more potent when assayed in the absence of DMSO

Aspects of the invention will now be exemplified by the following examples, which are understood to be illustrative and not limiting.

Example 6 gives preparation details of inventive embodiments with embodiments 3, 5, and 6 being the metallocomplexed compounds. Example 7 gives further details of our work to inhibit both HIV-1 and HIV-2 proteases, and summarizes some comparative work with cellular aspartyl proteases.

### EXAMPLE 1

### Synthesis of bis-glycol porphyrin dimethyl ester.

Dried protoporphyrin dimethyl ester (4.4 g) was dissolved in 1.6 L of dioxane and 3.0 mL of pyridine in the dark. This solution was thoroughly bubbled with argon to remove oxygen. Osmium tetroxide (4.0 g) was dissolved in 200 ml argon-degassed diethyl ether and added to the dioxane-porphyrin solution. The solution was well stirred under Ar in the dark for 24 hours. Sodium sulfite (8.8 g) was dissolved in 160 mL distilled, argon-degassed water and added to the solution. The reaction was heated to 75°C on a steam bath for 6 hours. The reaction solution was cooled to 53-55°C and filtered quickly through a 1.5 L medium fritted funnel and washed with a small portion of dioxane. Filtrate was evaporated *in vacuo* and then 200 mL and 750 mL of water was added with stirring to crystallize the product. The crystals were filtered through a 1.5 L medium fritted funnel and washed with 100 mL water. The filtered solid was suspended in 200 mL, 15% methanol/methylene chloride and 450 mL hexane added with stirring to complete crystallization. The procedure was repeated to remove impurities if necessary.

### EXAMPLE 2

### Synthesis of 2,4-bis-[α,β-(1,2-dicarbaclosododecaborane carboxy)ethyl]deuteroporphyrin (IX) dimethyl.

Carborane carboxylic acid was synthesized from 1,2-B₁₀C₂H₁₂ by the method of Zakharkin et al., Akad. Nauk SSSR, *Ser*. *Khim*., p. 1376 (1967) and Zakharkin et al., *Tet*. *Lett.,* p. 1147 (1964), incorporated by reference. Briefly, this method involves treatment of the o-carborane with one equivalent of n-butyl-lithium in benzene to produce the mono-lithio compound. This species is reacted with CO₂ to give the lithium salt of the carboxylate which gives the free carboxylic acid on acidification. Conversion to the acid chloride is also by the method of Zakharkin et al. using PCl₅ in toluene. Vacuum distillation of the reaction mixture gives the carborane carbonyl chloride.

The bis-glycol porphyrin prepared as described by Example 1 (500 mg, 0.75 mmol) was dissolved in dry methylene chloride (200 mL), and the solution bubbled with argon. To the above solution o-carboranyl acid chloride (690 mg, 3.341 mmol) was added. The solution was stirred, and 4-dimethyl aminopyridine (DMAP) 371 mg, 3 mmol) was added to the solution. The solution was stirred at room temperature for one hour and poured into water. The organic layer was separated, washed with dilute hydrochloric acid three times, saturated sodium bicarbonate (3x), water (2x), and dried over sodium sulfate. Unreacted carborane carboxylic acid was removed from the bicarbonate washes by acidification and extraction with hexane or diethyl ether. The solution was filtered and evaporated *in vacuo* to yield a crude product. Three spots were obtained on analytical TLC plate by developing with 100% methylene chloride. The top spot is the tetracarboranyl porphyrin dimethyl ester, and the two slower spots are tri- and di-carborane esters, respectively. The tetracarboranyl porphyrin dimethyl ester was separated by filtering the methylene chloride solution of the crude products through a silica gel pad. The first mobile band was obtained by washing with 100% methylene chloride (110 mL) and evaporating to dryness. Crystallization from methylene chloride-hexane gave 849 mg. Isolated yield was 80-85%. We coined "BOPP" as a shorthand designation for the inventive embodiment whose preparation has just been described (in the dimethyl ester form).

The conversion of bis-glycol to tetra-ester critically depends upon the use of p-dimethylaminopyridine (DMAP) as a hyper-acylation reagent and upon its stoichiometric relationship with bis-glycol and acid chloride. The rate of formation of tetra-ester product in the absence of nitrogen base is extremely slow, if it occurs at all. We have found that when pyridine or trimethylamine (two other frequently used acylation agents) were used rather than the DMAP reagent, then the rate of tetra-ester formation was very slow when compared to use of DMAP. However, when one wishes to prepare the diester form rather than the tetra-ester, then these other acylation agents (such as TEA) should be used rather than DMAP.

### EXAMPLE 3

### Synthesis of 2,4-bis-[α,β-(1,2-dicarbaclosododecaborane carboxy)ethyl]deuteroporphyrin (IX).

To a solution of the tetracarboranylporphyrin dimethyl ester prepared as described in Example 2 (300 mg, 0.224 mmol) in 100 mL ether was added 25% hydrochloric acid (100 mL). The solution was stirred at room temperature overnight. The solution was washed with copious water (to dilute acid). The ether layer was separated, dried over sodium sulfate and evaporated *in vacuo* to give the tetracarboranyl porphyrin diacid inventive embodiment we designate in shorthand as "BOPP free acid." Quantitative yield.

### EXAMPLE 4A

### Synthesis of 2,4-bis-[α,β-(1,2-dicarbaclosododecaborane carboxy)ethyl]deuteroporphyrin (X).

The tetracarboranyl porphyrin diacid, or "BOPP free acid" prepared as described in Example 3, (150 mg) was dissolved in 20 mL THF and 15 mL water was added. The solution was passed through 1x10 cm cation exchange resin, 200-400 dry mesh. The eluate was evaporated to increase the ratio of water (up to 60/40 water/THF) and passed through ion exchange resin again. The final eluate was evaporated to dryness *in vacuo.* The resulting dipotassium salt is well solubilized in water. We designated this inventive embodiment as "BOPP."

### EXAMPLE 4B

### Synthesis of 1,7 Isomer.

The 1,7 *closo*-carborane isomer of BOPP was prepared in the same manner using 1,7-carborane carbonyl chloride, but with the following difference. Thermal isomerization of the 1,2-carborane to the 1,7-carborane occurs at 450-500°C. See Grafstein and Dvorak, *Inorganic Chemistry,* 2:1128 (1963). Yields in this process approach 95% when a flow through a pyrolysis system is used in conjunction with an inert carrier gas. This approach is preferred for the conversion of ¹⁰B-enriched 1,2 isomer to the 1,7 isomer.

Other sterically hindered organic acid chlorides were used to incorporate desired R³ moieties in an analogous manner to Examples 1-4A. Characterization in all cases was by mass spectrometry, proton magnetic resonance, and UV-visible spectroscopy.

### EXAMPLE 5

Evidence in support of the structures prepared in Examples 2-4A came from mass spectrometric and spectroscopic sources. The LSIMS mass spectrum of BOPP-dimethyl ester in a tetraethylene glycol matrix shows a molecular ion cluster (MH⁺) at nominal mass 1341 corresponding to the formula C₄₈H₈₃B₄₀N₄O₁₂. The shape of the theoretical molecular ion cluster of this formula is nearly identical to the 15-peak ion cluster observed at 1341. Similarly, LSIMS of the BOPP free acid produces a molecular ion cluster at nominal mass 1321 corresponding to the formula C₄₆H₇₉B₄₀N₄O₁₂ and whose shape is almost identical to the theoretical molecular ion cluster. In both mass spectra, four successive losses of B₁₀H₁₁C₃O₂ fragments are observed corresponding to loss of the carborane carboxylate. The visible spectrum of BOPP-dimethyl ester in CH₂Cl₂ (10 *µ*M) consists of peaks at 404 (Soret) 502, 536, 572, and 624 nm.

A summary of the 300 MHz proton N.M.R. data for BOPP-dimethyl ester is presented in Table 1.

**TABLE 1***

| δ | multiplicity | assignments |
|---|---|---|
| 10.27, 10.23, 10.16, 10.14 | s, 4H | meso H |
| 7.68 | m, 2H | α CH |
| 5.68; 4.99 | m, 4H | β CH₂ |
| 4.41 | m, 4H | Por-CH₂ |
| 4.17; 4.10 | s, 4H | carborane CH |
| 3.81; 3.80 | s, 6H | CO₂CH₃ |
| 3.68; 3.64 3.63; 3.61 | s, 12H | β-CH₃ |
| 3.30 | m, 4H | -CH₂CO₂R |
| -3.65 | s, 2H | NH |
| ^{*} N.M.R. spectrum was recorded in CDCl₃ (ca. 5x10⁻³ M) at 300 MHz at ambient temperature with TMS internal reference. | | |

The lack of a molecular C₂ axis is clearly demonstrated by the presence of four, distinct resonances for the meso-H and β-CH₃ groups. This makes difficult a detailed analysis of the spectrum, especially the conformation of the carboranyl ester-bearing side chains. Nevertheless, several features are apparent. At least two distinct and equivalent carborane CH environments are present, perhaps a reflect:ion of the primary and secondary alcohol ester functions. Three resonances are assigned to the two-carbon side chain protons, a chiral methine (H_{α} ), and pro-chiral methylene (H_{β}). The H_{α} assigned to 7.68 ppm is strongly deshielded by virtue of its being bound to a carbon bearing two strongly deshielding groups: the porphyrin (ring current) and carboranyl acyl (σ effects). The two H_{β} resonances have significant (0.7 ppm) chemical shift differences which might arise if the most stable configuration forces one of the H_{β} protons into a position over the porphyrin ring when it is subject to ring current deshielding relative to the other.

There are three possible configurations for the groups bound to the ethyl side chain, two gauche and one anti. Computer modeling based on the crystal structure of mesoporphyrin IX dimethyl ester suggests that the anti-configuration should offer the least amount of steric hindrance for all functional groups. The α-carborane ester points down at an angle of about 45° and away from the porphyrin while the β-ester points up and over the porphyrin. This configuration also places one of the pro-chiral H_{β} protons in a pocket formed by the porphyrin plane, a meso-H and the O_{α}, giving rise to its potential deshielding relative to the other H_{β}. However, one gauche configuration also provides some side chain flexibility and deshielding of one H_{β} from a similar pocket. In the remaining gauche form, all J-J coupling constants should be large and modeling indicates significant steric hindrance for the O_{β}.

### EXAMPLE 6

In a manner analogous to that described by the just described examples, a number of inventive embodiments and comparative compounds were prepared where Table 2 gives the R, R¹, and R⁴ moieties.

**TABLE 2**

| Inventive Embodiment | R | R¹ | R⁴ |
|---|---|---|---|
| 1 | OCOB₁₀H₁₁C₂ | Same as R | H₂ |
| 2 | H | OCOB₁₀H₁₁C₂ | H₂ |
| 3 | OCOB₁₀H₁₁C₂ | Same as R | Mn(III) |
| 4 | OCOB₁₀H₁₄C₃ | Same as R | H₂ |
| 5 | OCOB₁₀H₁₁C₂ | Same as R | Cu(II) |
| 6 | OCOB₁₀H₁₁C₂ | Same as R | Co(II) |
| 7 | meta-OCOB H C | Same as R | H₂ |

| Comparative Embodiment | | Same as R | H₂ |
|---|---|---|---|
| 8 | OCO(adamantoyl) | Same as R | H₂ |
| 9 | OCOp-((CH₃)₂N)benzoyl | Same as R | H₂ |
| 10 | OCOC₆H₅ | Same as R | H₂ |
| 11 | OCO(β-naphthoyl) | Same as R | H₂ |
| 12 | OH | OH | H₂ |

Compound 2 was prepared in the dimethyl ester form as described by Example 2 and converted to the free acid as by Example 3.

Compounds 3, 5, and 6 are metalloporphyrin derivatives of BOPP prepared by the general procedure outlined below. BOPP dimethyl ester (130 mg/0.099 mmol) was dissolved in glacial acetic acid (9 ml) and pulverized anhydrous manganese (II) acetate (50 mg; 0.289 mmol) was added. The vessel was loosely stoppered and the solution stirred in the dark at 20°C. The course of reaction was followed spectrophotometrically by observing the disappearance of the red fluorescence characteristic of free base porphyrin. When all traces of this red fluorescence had disappeared (20 h), ether (125 ml) was added to the solution. The other solution was washed with an equal volume of water (3x), dried over sodium sulfate, and evaporated *in vacuo*. The solid product was redissolved in ether (30 ml) and 25% aqueous HCl (30 ml) added. After stirring overnight at 20°C, the mixture was poured into ether (200 ml) and washed four times with water (500 ml). The ether layer was again dried, filtered, and evaporated *in vacuo* to yield pure product (166 mg, 75.1% yield).

The water soluble Mn(III) porphyrin dipotassium salt is estimated to contain 5% water by weight. The absorption spectrum of this compound showed no evidence of free-base porphyrin and consisted of peaks at 368 nm (log ε = 4.82), 418 nm (sh) (4.36), 460 nm (4.60), 546 nm (3.98), and 579 nm (sh) (3.82). The Cu(II) (compound 5) and Co(II) (compound 6) derivatives were prepared in a similar fashion to 3 using the appropriate metal salt. Their UV-Vis absorption spectra were similar to that of compound 3 and were characteristic of metalloporphyrins of the hematoporphyrin class. As with 3, no evidence of unmetallated free base porphyrin was observed in the UV-Vis spectra.

The twelve compounds summarized in Table 2 were then tested for specific inhibition of HIV-1 protease as follows.

### EXAMPLE 7

Recombinant Protein Preparation. Recombinant HIV-1 protease (HIV-1 PR) was expressed and purified from *E. coli* strain D1210 using the pSOD/PR179 vector. Recombinant HIV-2 protease (HIV-2 PR) and SIV protease (SIV PR) were expressed and purified from strain X90 using pTacTac vectors.

Enzyme Assays. HIV-1, HIV-2, and SIV PRs were assayed against the decapeptide corresponding to the HIV-1 PR C-terminal autoprocessing site, by means of a discontinuous HPLC assay. Recombinant human renin and porcine pepsin were obtained and assayed using standard conditions. Bovine cathepsin D and its chromogenic substrate were purchased from Sigma. The enzyme was assayed by standard methods.

Stock solutions of the porphyrin derivatives (1 *µ* M-10mM) in 100% DMSO were used in IC₅₀ determinations. Since many of the derivatives were insoluble in buffer alone, including the free acid forms of the carborane compounds, DMSO was used to increase solubility and allow a comparison of the binding affinity. Compounds were added to buffer solutions containing additional DMSO to give a final concentration of 5%. Control reactions contained 5% DMSO only. Enzymes were preincubated with inhibitor for 1 minute at 25°C, followed by addition of substrate to initiate the reaction. As earlier noted, assays were also carried out in the absence of DMSO, when possible. Dipotassium salts of several of the inhibitors were soluble in aqueous solution and were assayed in the absence of DMSO for K₁ and IC₅₀ determinations. These IC₅₀ data (*µ*M) are as follows:

| Inventive | IC₅₀ *µM* | |
|---|---|---|
| Embodiment | HIV-1 PR | HIV-2 PR |
| 1 | 0.05 | 0.230 |
| 3 | 0.10 | 0.70 |
| 4 | 0.90 | 0.550 |
| 5 | 0.725 | 0.470 |

The salt dependency of inhibition of HIV-1 PR by inventive embodiment 3 was also tested by varying the [NaCl] from 1M to 0.3 M in the assay buffer, which showed the inhibition was not highly dependent on salt concentration.

*Ex vivo* Assay of HIV-1 Polyprotein Processing. COS A6 cells were the result of stable integration of the vector HIV-gpt by transfection of COS-7 cells. This SV40-based vector consists of the HIV-1 HXB2 strain proviral genome where the gp160 sequences were replaced by the guanidyl phosphate ribosyltransferase (gpt) gene. Following transfection, the cells were placed under selection for gpt expression by adding mycophenolic acid to the media. Surviving cells were later cloned and assayed for expression of gag p24 protein by ELISA and Western blots of culture supernatants and whole cell extracts. The cloned COS A6 cell line was maintained in DME H21 supplemented with 10% dialyzed and refiltered fetal calf serum (FCS), antibiotics (100 U/ml penicillin G, 100 *µ*g/ml streptomycin sulfate), hypoxanthine (14 *µ*g/ml), xanthine (250 *µ*g/ml), and mycophenolic acid (25 *µ*g/ml), at 37°C and 5% CO₂.

MTT Cell Viability Assay. Cell survival was assessed by a quantitative colorimetric assay. The tetrazolium salt 3-(4,5-dimethylthiazol-2-yl)2,5-diphenyltetrazolium bromide (MTT) is cleaved by dehydrogenases in active mitochondria of living cells to yield a change in color from yellow to purple. To obtain the LD₅₀ values for each compound, the concentration was determined at which the absorbance at 550-570 nm was half of that for the untreated cells. Compounds were tested in duplicate, in serial dilutions generally ranging from 10-250 *µ*M concentrations in the presence of 0-0.1% DMSO. MTT assays were carried out in both the presence and absence of 10% FCS.

Effect of Albumin on Efficacy of Boronated Porphyrins. Since porphyrin compounds are known to bind to albumin, we tested the effect of fetal calf serum on the ability of inventive embodiment 1 to inhibit HIV-1 PR *in vitro.* We found that incubation with 0.1% FCS decreased inhibition of the protease normally seen in the presence of 50 nM inventive embodiment 1 was more than 50%. Since growth and viability of COS A6 cells requires at least 2% FCS in culture medium for incubations longer than 1 hour, we chose to test the effect of porphyrin compounds on polyprotein processing using a pre-adsorption step in Dulbecco's phosphate-buffered saline (PBS), as described below.

Treatment of COS A6 Cells with Boronated Porphyrins. Cells were grown to 75% confluency in 10 cm dishes and rinsed with warm PBS. The appropriate dilution of the compounds was prepared by adding 2.5 ml of warm PBS to 2.5 *µ*l of a stock solution prepared in neat DMSO. This material was carefully added to the cells for a 15 minute incubation. Next 2.5 ml of DME media containing 10% FCS and the appropriate compounds were added for 3.75 hours, after which time the culture supernatant was removed for isolation of viral capsids.

Viral Capsid Isolation by Ultracentrifugation. The culture supernatants were spun 10 minutes at 3,000 rpm to remove any precipitate or cell debris. The resulting supernatant was layered over a cushion consisting of 75 *µ*l of 60% sucrose in PBS overlayed with 4 ml of 20% sucrose in PBS. The sample was spun in an SW51 Beckman rotor for 1.5 hours at 35K rpm and 4°C. Since viral capsids band at the interface of the 20 and 60% sucrose solutions, the bottom 500 *µ*l of solution were collected.

p24 Core Antigen ELISA Assay. An ELISA kit (catalog # NEK-060) purchased from NEN/Dupont was used to determine the amount of p24 present in the viral capsid samples. This assay specifically detects p24 but not its precursors. The assay was carried out using the manufacturer's instructions. Values obtained were used to calculate the *ex vivo* IC₅₀, i.e., concentration of compound that reduces the amount of detectable p24 antigen by 50%.

The results of these studies are summarized by Table 3.

**TABLE 3**

| Inventive Embodiment | IC₅₀ *in vitro*^{a} HIV-1 | *µ*M HIV-2 | LD₅₀^{b} *µ*M |
|---|---|---|---|
| 1 | 0.185 | 0.70 | 25 |
| 2 | 0.275 | 1.0 | 75 |
| 3 | 0.40 | 1.2 | 70 |
| 4 | 0.70 | 1.55 | 25 |
| 5 | 0.975 | 2.2 | 80 |
| 6 | 2.25 | 1.5 | 150 |
| 7 | 12 | 11 | 250 |

| Comparative Embodiment | | | |
|---|---|---|---|
| 8 | 5 | 13 | 250 |
| 9 | 7 | 18 | >250 |
| 10 | 14 | 30 | 250 |
| 11 - | 14 | 23 | 250 |
| 12 | 280 | 480 | 250 |

| | | | |
|---|---|---|---|
| ^{a} Determined in the presence of 5% DMSO since as previously noted, some of the derivatives were insoluble in buffer alone; however, as earlier shown for inventive embodiments 1, 3, 4, and 5, better inhibition, particularly for inventive embodiment 1, was obtained in another series of experiments where assays were conducted in the absence of DMSO. | | | |
| ^{b} Cytotoxicity towards COS A6 cells cultured in the absence of FCS. | | | |

As exemplified and illustrated by the data of Table 3, the tetrakiscarborane carboxylate ester of 2,4-(α,β-dihydroxylethyl)deudero-porphyrin IX (that is, inventive embodiment 1) was shown to inhibit HIV-1 and HIV-2 proteases with IC₅₀ values of 50 and 230 nM, respectively. This particularly preferred embodiment has at least a sixty-fold greater affinity for HIV-1 PR than for cellular aspartyl proteases, as is shown by Table 4, which data was collected in the absence of DMSO.

**TABLE 4**

| IC₅₀ for Inventive Embodiment 1 on Viral and Cellular Aspartyl Proteases | |
|---|---|
| Protease | IC₅₀, *µ*M |
| Renin | 3 |
| Cathepsin D | 10 |
| Pepsin | 4 |
| HIV-1 | 0.05 |
| HIV-2 | 0.23 |
| SIV | 0.25 |

Figure 1 is a Dixon plot of inventive embodiment 5 (which is a representative example of the other embodiments) in inhibition of HIV-1 protease, where purified HIV-1 protease (6 x 10⁻¹ mg/ml) was incubated with the inventive inhibitor in 50 mM sodium acetate buffer, pH 5.5, containing 1 mM dithiothreitol, 1 mM EDTA, and 1 M NaCl. After 1 minute, the substrate peptide was added to give the final substrate concentrations shown. The assay solutions were incubated for 30-45 minutes at 37° and enzyme activity was determined by quantitation of the hydrolysis products on HPLC.

The importance of the Fig. 1 results are in showing the mode of binding by the embodiment to the enzyme. Thus, the inventive embodiment binds to the active site of the protease.

Cytotoxicity of compounds (LD₅₀) and their ability to inhibit capsid protein processing ex vivo during 4 hour incubations (IC₅₀) were studied. A plasmid which encodes the HIV-1 proviral genome, with the exception of the gp 150 envelope protein, was stably introduced into the monkey cell line COS 7. Cloned progeny, COS A6 cells, constitutively release viral capsids into the media. Inhibition of polyprotein processing was determined by measuring the amount of p24 present in the viral capsid samples with an ELISA assay. The decrease in the amount of detectable p24 antigen correlated with a specific inhibition of HIV PR activity, judged by the accumulation of capsid precursor in conjunction with a disappearance of the p24 mature protein band in Western blots. The MTT stain assay was used to obtain LD₅₀ values for all the compounds tested.

We have observed that the presence of albumin prevents the ability of the compounds to inhibit HIV-1 PR during the short-term incubations (0.25-4 hour); therefore, the IC₅₀ values were determined in the presence of a reduced concentration of fetal calf serum (FCS). However, COS A6 cells require 10% FCS for optimal growth and viability and certain porphyrin derivatives show cytotoxicity towards COS A6 cells at <100 *µ*M concentrations when cultured in the absence of FCS. Cytotoxicity is reduced by culturing COS A6 cells in 10% FCS. Under these conditions, the LD₅₀ for embodiments 1-11 is >250 *µ*M. Compound 1 has similar LD₅₀ values in C6 glioma and V79 CHO cells (100-125 *µ*M and ≥150 *µ*M, respectively) in the presence of FCS, as measured by standard colony survival techniques. Moreover, embodiment 1 is tolerated in mice at doses as high as 200 mg/kg, with no apparent signs of morbidity or mortality of animals. Mice receiving embodiment 1 at 100 mg/kg by i.v. bolus are exposed to peak plasma concentrations of approximately 900 *µ*M. The antagonistic effect of albumin in cell culture may not reflect the situation in whole animals, where serum proteins can bind porphyrins and deliver them to various tissues rather than sequestering them.

Inventive embodiment 1 is approximately 5-fold more effective for HIV-1 PR than for the closely related HIV-2 and SIV PRs, and at least 60-fold more inhibitory for HIV-1 PR compared to cellular aspartyl proteases (as shown in Table 4).

In sum, we have found that certain substituted porphyrins are inhibitors of retroviral aspartyl proteases, such as HIV-1 PR and HIV-2 PR, and inhibit polyprotein processing in cell culture, yet inhibition is selective over cellular aspartyl proteases.

It is to be understood that while the invention has been described above in conjunction with preferred specific embodiments, the description and examples are intended to illustrate and not limit the scope of the invention, which is defined by the scope of the appended claims.

## Claims

1. A porphyrin-based compound having the structure: 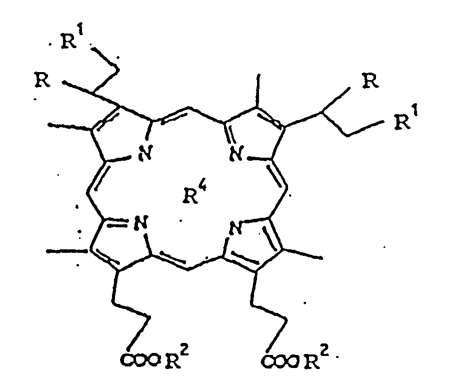 where each of R and R¹ is selected from -H, -OH, or 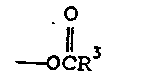 and at least one of R and R¹ is 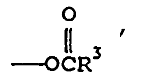 R³ is a carborane, R² is -H, an alkyl, or an aryl, having 1 to 7 carbon atoms, or a physiologically acceptable salt, and R⁴ is H, a metal, or a transition metal.

2. The porphyrin-based compound according to claim 1, wherein R⁴ is manganese, cobalt, or copper.

3. Use of the compound according to claim 1 for the preparation of a medicament effective for the treatment of diseases caused by a virus that replicates through use of a protease comprising:
administrating said porphyrin compound; or a physiologically acceptable salt thereof to a patient in a therapeutic amount effective to inhibit the protease.

4. The use according to claim 3, wherein the carborane is a closo-carborane.

5. The use according to claim 3, wherein the metal or transition metal of R⁴ is manganese, cobalt, or copper.

6. The use according to claim 3, wherein the porphyrin compound is administrated in a pharmacologically acceptable carrier.

7. The use according to claim 6, wherein the porphyrin compound is solubilized in the carrier.

8. The use according to claim 6, wherein the carrier is aqueous based.

9. The use according to claim 3, wherein the porphyrin-based compound is water-soluble.

10. The use according to claims 1 or 6, wherein administration is oral or by injection.

11. The use according to claim 10, wherein administration is by dosages up to 200 mg/kg.

## Patentansprüche

1. Verbindung auf Basis von Porphyrin mit der folgenden Struktur: 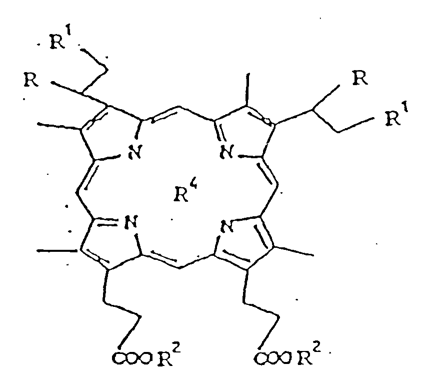 in der R und R¹ jeweils ausgewählt sind aus -H, -OH oder 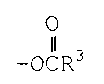 und wenigstens ein Rest aus R und R¹ 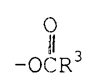 ist, R³ ein Carboran ist, R² -H, eine Alkyl- oder eine Arylgruppe mit 1 bis 7 Kohlenstoffatomen oder ein physiologisch annehmbares Salz ist, und R⁴ H, ein Metall oder ein Übergangsmetall ist.

2. Verbindung auf Basis von Porphyrin nach Anspruch 1, wobei R⁴ Mangan, Kobalt oder Kupfer ist.

3. Verwendung der Verbindung nach Anspruch 1 für die Herstellung eines Medikaments, das wirksam ist für die Behandlung von Krankheiten, die durch einen Virus verursacht sind, der sich durch Einsatz einer Protease repliziert, wobei die Verwendung aufweist:
Verabreichung der Porphyrinverbindung; oder eines physiologisch annehmbaren Salzes von dieser an einen Patienten mit einer therapeutischen Menge, die wirksam ist, um die Protease zu inhibieren.

4. Verwendung nach Anspruch 3, wobei das Carboran ein *closo*-Carboran ist.

5. Verwendung nach Anspruch 3, wobei das Metall oder das Übergangsmetall von R⁴ Mangan, Kobalt oder Kupfer ist.

6. Verwendung nach Anspruch 3, wobei die Porphyrinverbindung in einem pharmakologisch annehmbaren Träger verabreicht wird.

7. Verwendung nach Anspruch 6, wobei die Porphyrinverbindung in dem Träger gelöst ist.

8. Verwendung nach Anspruch 6, wobei der Träger auf Wasser basiert.

9. Verwendung nach Anspruch 3, wobei die Verbindung auf Basis von Porphyrin wasserlöslich ist.

10. Verwendung nach Anspruch 1 oder 6, wobei die Verabreichung oral oder durch Injektion erfolgt.

11. Verwendung nach Anspruch 10, wobei die Verabreichung mit Dosismengen von bis zu 200 mg/kg erfolgt.

## Revendications

1. Composé à base de porphyrine, ayant la structure : 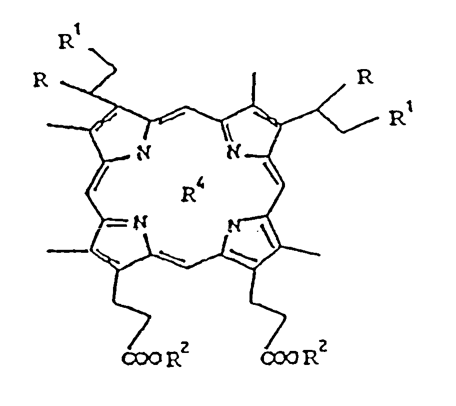 dans laquelle chacun des radicaux R et R¹ est choisi parmi -H, -OH ou 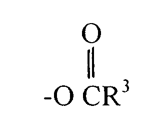 et au moins l'un des radicaux R et R¹ est 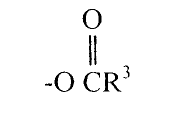 R³ est un carborane, R² est -H ou un groupe alkyle ou aryle ayant de 1 à 7 atomes de carbone, ou un sel physiologiquement acceptable, et R⁴ est H, un métal ou un métal de transition.

2. Composé à base de porphyrine selon la revendication 1, dans lequel R⁴ est le manganèse, le cobalt ou le cuivre.

3. Utilisation du composé selon la revendication 1 pour préparer un médicament efficace pour le traitement de maladies provoquées par un virus qui se réplique par utilisation d'une protéase, et qui comprend :
l'administration dudit composé de porphyrine, ou d'un sel physiologiquement acceptable de ce dernier, à un patient et en une quantité thérapeutique permettant l'inhibition de la protéase.

4. Utilisation selon la revendication 3, dans laquelle le carborane est un closo-carborane.

5. Utilisation selon la revendication 3, dans laquelle le métal ou le métal de transition de R⁴ est le manganèse, le cobalt ou le cuivre.

6. Utilisation selon la revendication 3, dans laquelle le composé de porphyrine est administré dans un excipient acceptable d'un point de vue pharmacologique.

7. Utilisation selon la revendication 6, dans laquelle le composé de porphyrine est solubilisé dans l'excipient.

8. Utilisation selon la revendication 6, dans laquelle l'excipient est à base aqueuse.

9. Utilisation selon la revendication 3, dans laquelle le composé à base de porphyrine est soluble dans l'eau.

10. Utilisation selon les revendications 1 ou 6, dans laquelle l'administration s'effectue par voie orale ou par injection.

11. Utilisation selon la revendication 10, dans laquelle l'administration s'effectue à des posologies allant jusqu'à 200 mg/kg.
